Europäisches Patentamt

European Patent Office

Office européen des brevets

(19)

(11) Numéro de publication: **0 285 470**
**A1**

## (12) DEMANDE DE BREVET EUROPEEN

(21) Numéro de dépôt: 88400483.9

(22) Date de dépôt: 02.03.88

(51) Int. Cl.⁴: **A 61 M 16/00**
G 01 L 9/00

(30) Priorité: 05.03.87 FR 8702978

(43) Date de publication de la demande:
05.10.88 Bulletin 88/40

(84) Etats contractants désignés:
DE ES FR GB IT NL SE

(71) Demandeur: L'AIR LIQUIDE, SOCIETE ANONYME POUR
L'ETUDE ET L'EXPLOITATION DES PROCEDES
GEORGES CLAUDE
75, Quai d'Orsay
F-75321 Paris Cédex 07 (FR)

(72) Inventeur: Champain, Roger
2, rue Ferme de l'Hopital
F-78350 Les-Loges-en-Josas (FR)

Theurant, Gilbert
47, rue Marie Sorin-Defresne
F-794400 Vitry-sur-Seine (FR)

(74) Mandataire: Leclercq, Maurice et al
L'AIR LIQUIDE SOCIETE ANONYME POUR L'ETUDE ET
L'EXPLOITATION DES PROCEDES GEORGES CLAUDE
75, Quai d'Orsay
F-75321 Paris Cedex 07 (FR)

(54) **Procédé et dispositif d'alimentation en oxygène respiratoire.**

(57) L'invention concerne l'alimentation en air suroxygéné d'un patient pendant une partie de la phase d'inspiration.

L'électrovanne d'alimentation (4) est commandée à l'ouverture dès que le récepteur (23) recueille un signal de dépression du à un début d'inspiration, produisant une tension en (31) comparée à une tension de référence correspondant à la pression atmosphérique précédemment mémorisée. La durée d'injection comptée en (45) est proportionnelle à la durée de non-injection comptée en (48) qui elle-même est dépendante de la durée de la phase expiratoire.

EP 0 285 470 A1

## Description

### PROCEDE ET DISPOSITIF D'ALIMENTATION EN OXYGENE RESPIRATOIRE"

La présente invention concerne l'alimentation additionnelle en oxygène respiratoire d'un patient, où l'on assure la dite alimentation en complément de l'alimentation naturelle en air atmosphérique seulement pendant une partie de la phase inspiratoire, qui est initiée par la détection, le cas échéant anticipée, du début d'une phase inspiratoire par rapport à la pression atmosphérique dans une chambre détectrice du type à baudruche et capteur électronique.

Le brevet français 84.17.236 décrit un capteur électronique avec une baudruche équipée d'une languette s'interposant sur le parcours optique lors d'une dépression inspiratoire, ce qui a pour effet de commander, pendant un laps de temps déterminé, l'ouverture d'une électrovanne d'alimentation en gaz respirable. Ce mode opératoire est susceptible d'une bonne sensibilité sous réserve d'un réglage fin en position des différentes pièces coopérantes, réglage qui, en général doit être renouvellé de temps en temps.

La présente invention a pour objet d'améliorer ce type d'alimentation en prévoyant des dispositions qui permettent une sensibilité accrue sans aucune obligation ultérieure de réglage en cours de fonctionnement et qui est doté d'un étalonnage automatique.

Selon une caractéristique essentielle du procédé d'alimentation selon la présente invention, on assure la détection opto-électronique par un capteur du genre à réflexion sur un réflecteur plaqué contre une paroi de baudruche qui est à l'opposé de la paroi de fixation de la dite baudruche, et on utilise la variation de courant du capteur lors d'une réduction de la distance capteur-baudruche due à une dilatation de la baudruche résultant d'un début, le cas échéant anticipé, d'une phase inspiratoire pour assurer une commande prédéterminée de l'alimentation en gaz respiratoire ; selon une particularité essentielle, ou assure, pendant chaque phase expiratoire, une mémorisation d'une tension de référence de sortie de capteur représentative de la pression atmosphérique et on déclenche l'alimentation en gaz respirable par comparaison de cette tension de référence avec un signal de tension représentatif d'une baisse de pression inspiratoire, signalant la fin d'une phase expiratoire ou le début d'une phase inspiratoire.

De la sorte, grâce à cette comparison répétée entre deux tensions, dont la différence marque le début d'une phase inspiratoire, on peut être assuré d'un fonctionnement correct, même si l'appareillage mis en oeuvre subit dans le temps une évolution de ses propres caractéristiques.

L'invention concerne également un dispositif d'alimentation additionnelle en oxygène respirable, du genre comportant une conduite d'alimentation d'une lunette respiratoire ou d'une sonde trachéale, incorporant une électrovanne à trois voies, dont la troisième voie est raccordée à la troisième voie d'une électrovanne de mise à l'air atmosphérique d'un détecteur à capteur opto-électronique associé à une baudruche, des moyens de commande cyclique des dites vannes d'alimentation en oxygène respirable et de mise à l'air, et ce dispositif est caractérisé en ce que le capteur opto-électronique est du type à réflexion sur un réflecteur plaqué contre une paroi de baudruche, qui est opposée à une paroi de fixation de baudruche. Selon une forme préférentielle de mise en oeuvre, les moyens de commande cyclique des vannes d'alimentation en oxygène respirable et de mise à l'air comprennent un moyen d'élaboration d'une tension électronique représentative de la pression dans la chambre du détecteur, un moyen de mémorisation de la dite tension pendant la mise à l'air du détecteur, ou tension de référence, des moyens de comparaison entre la tension de référence mémorisée et la tension instantanée représentative de la pression dans la chambre du détecteur, les dits moyens de comparaison assurant, lors d'une différence avec la tension de référence signalant le début le cas échéant anticipé d'une phase inspiratoire la commande d'ouverture de la vanne d'alimentation en oxygène respirable et une commande différée, au-delà de la phase d'alimentation d'ouverture de la vanne de mise à l'air libre du détecteur.

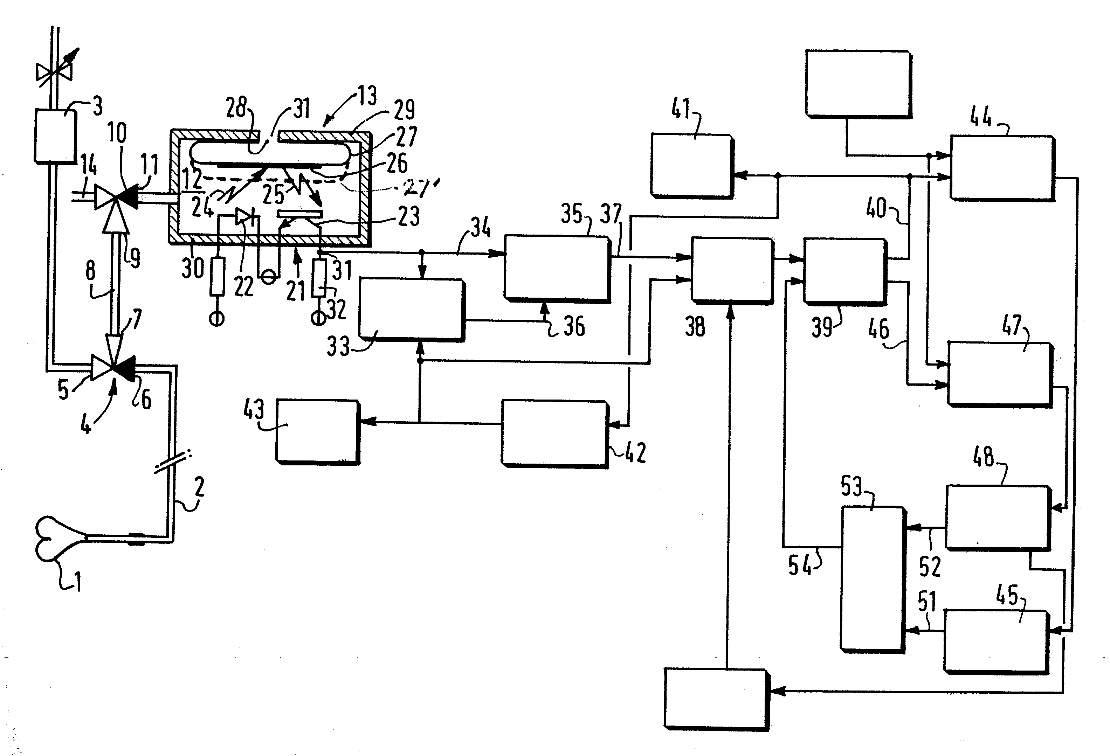

Les caractéristiques et avantages de l'invention ressortiront d'ailleurs de la description qui suit, à titre d'exemple, en référence au dessin annexé qui représente, à titre d'exemple, une forme de réalisation schématique de l'invention.

Une lunette respiratoire 1 est raccordée par une conduite 2 à une capacité-tampon 3 avec interposition d'une électrovanne d'alimentation 4 à trois voies 5, 6 et 7, tandis que la capacité-tampon 3 est elle-même raccordée, par un orifice calibré réglable, à un concentrateur non représenté, qui assure une concentration en oxygène de l'air atmosphérique. De tels concentrateurs sont avantageusement du type à adsorption.

L'électrovanne 4 a deux positions opératoires, à savoir :
- une position d'alimentation, dans laquelle les voies 5 et 6 communiquent l'une avec l'autre, tandis que la voie 7 est obturée : c'est la position d'alimentation en gaz respirable suroxygéné de la lunette 1 par la conduite 2 à partir de la capacité-tampon 3. ;
- une position d'attente, dans laquelle les voies 6 et 7 communiquent l'une avec l'autre, tandis que la voie 5 est obturée. C'est une position qui transmettra, comme on va le comprendre maintenant, le signal de début d'inspiration ou de fin d'expiration.

En effet, la voie 7 de l'électrovanne 4 est raccordée par une conduite 8 à une troisième voie 9 d'une électrovanne de détection 10, dont une première voie 11 communique avec une chambre interne 12 d'un détecteur opto-électronique 13, tandis qu'une seconde voie 14 de l'électrovanne 10 débouche à l'air libre.

L'électrovanne 10 a deux positions opératoires, à savoir :
- une position d'attente du signal de fin d'expiration ou de début d'inspiration, dans laquelle les voies 9 et 11 communiquent l'une avec l'autre, en attente du dit signal qui est une dépression véhiculée depuis la lunette de patient 1 via la conduite 2, les voies 6 et 7 de l'électrovanne 4, les voies 9 et 11 de l'électrovanne 10 jusqu'à la chambre 12 du détecteur 13 ; dans cette position la voie 14 est obturée ;

- une position de reprise de la pression atmosphérique, dans laquelle les voies 11 et 14 communiquent l'une avec l'autre, tandis que la voie 9 est obturée ; dans cette position, la chambre 12 du détecteur 13 est portée à la pression atmosphérique.

Dans la chambre 12 du détecteur 13 sont placés les organes de détection proprement dits, à savoir :

- en position inférieure, un capteur à réflexion 21 comportant un émetteur optique 22 associé à un récepteur optique 23, la faisceau optique étant émis en 24 dans le sens ascendant, tandis que ce même faisceau réfléchi en 25 par un réflecteur plan 26 est reçu par le récepteur 23 en fin de son parcours retour descendant.

Le réflecteur plan 26 est solidaire du fond d'une baudruche 27, qui est un sac formé d'une paroi fine, souple et très légère, et présentant une ouverture unique 28.

Cette baudruche 27 est fixée de façon étanche contre une paroi supérieure 29 du détecteur 13, de façon que le réflecteur plan 26 se présente en position horizontale parallèlement à la paroi de fond 30 du détecteur 13, tandis que l'ouverture 28 se présente en regard d'une ouverture 31 de la chambre de détecteur 12, de sorte que l'intérieur de la baudruche 27 est toujours à la pression atmosphérique.

Lorsque l'électrovanne 10 est en position de reprise de la pression atmosphérique, via les voies 8 et 11, la baudruche 27 est dans la position indiquée en trait plein.

Lorsque l'électrovanne 10 est en position d'attente d'un signal d'inspiration, via les voies 9 et 11 de l'électrovanne 4 et les voies 6 et 7 de l'électrovanne 4, la baudruche 27 conserve la même configuration aussi longtemps que le signal de dépression du à une fin d'expiration ou à une début d'inspiration n'est pas parvenu dans la chambre 12, mais dès que la chambre 12 subit la baisse de pression, la baudruche 27 prend presque instantanément une forme plus allongée 27', du fait de la pression plus élevée qui règne à son intérieur.

Dans la position retractée 27 de la baudruche, le parcours du signal optique est le plus long et le capteur 23 recueille un flux optique relativement faible.

Dans la position allongée 27' de la baudruche 27, le parcours du signal optique est plus court et le capteur 23 recueille un flux optique relativement fort, car il y a moins de déperditions optiques.

C'est cette différence des flux optiques recueillis par le récepteur 23 qui assure le fonctionnement régulée de l'alimentation en gaz respirable.

Le signal repris sur le capteur est appliqué à la borne 31 d'une résistance 32 déterminant à ce niveau une tension.

La tension à la borne 31 est appliquée à un dispositif de mémorisation 33, et à une entrée 34 d'un comparateur analogique 35, dont l'autre entrée 36 reçoit un signal provenant du dispositif de mémorisation 33.

Le signal à la sortie 37 du comparateur analogique 35 est appliqué à une porte électronique 38 de commande d'un organe bistable 39 dont une sortie 40 est raccordée à un circuit de commande 41 de l'électrovanne 4 d'une part, à un dispositif de temporisation 42 et de là au dispositif de mémorisation 33 et à un dispositif de commande 43 de l'électrovanne 10 d'autre part, et enfin à une porte électronique 44 de commande d'un compteur électronique 45.

Une seconde sortie 46 de l'organe bistable 39 est raccordée à une porte électronique 47 de commande d'un compteur 48, dont la vitesse de comptage est notablement plus faible que la vitesse de comptage du compteur 45.

Les signaux de comptage des compteurs 45 et 48 sont appliqués aux entrées 51, 52 d'un comparateur analogique 53, dont le signal à une sortie 54 est appliqué à une seconde entrée de l'organe bistable 39.

Un dispositif à horloge 55 assure le fonctionnement propre des compteurs 45 et 48.

L'installation qui vient d'être décrite opère de la façon suivante :

Aus repos, lorsque la lunette 1 n'est pas raccordée au patient, l'électrovanne 4 assure l'arrêt de l'alimentation : voie 5 obturée, voies 6 et 7 raccordées, tandis que l'électrovanne 10 est en fonction d'attente du signal de fin d'expiration ou de début d'inspiration : voie 14 obturée, voies 9 et 11 raccordées. Il en résulte que la chambre 12 du détecteur 13 est raccordée à la lunette 1 et comme celle-ci n'est pas utilisée, la chambre 12 est à la pression atmosphérique et la baudruche 27 en position rétractée. La tension à la borne 31 est représentative de la pression atmosphérique, c'est la tension de référence qui peut être enregistrée dans le dispositif de mémorisation 33.

A la mise en place de la lunette 1, l'effort inspiratoire du patient transmis par l'électrovanne 4 (via les voies 6 et 7) et l'électrovanne 10 (via les voies 9 et 11) provoque une dépression dans la chambre 12 du détecteur 13, assurant l'expension immédiate de la baudruche 27 vers l'état 27'. Le récepteur 21 enregistre un flux optique plus important, de sorte, que le potentiel à la borne 31 varie dans un sens qui fait passer à une polarité positive la sortie 37 du comparateur analogique 35 précédemment à une polarité négative. Ce changement de polarité modifie l'état de l'organe bistable 39 dont la sortie 40 devient positive, ce qui à pour effet :

- d'assurer via le dispositif 41 la commande d'ouverture de l'électrovanne d'alimentation 4 (voies 5 et 6 en communication et voie 7 obstruée), ce qui envoie du gaz respirable vers le patient en cours d'inspiration ;

- d'assurer la commande du dispositif de temporisation 42 qui provoque immédiatement le fonctionnement du dispositif de commande 43 de l'électrovanne 10 pour mettre en communication les voies 11 et 14, de sorte que la chambre 12 du capteur 13 revient à la pression atmosphérique ;

- simultanément, le dispositif de mémorisation 33 est mis en fonctionnement, ce qui entraîne la mémorisation d'une tension atmosphérique instantanée juste avant une prochaine inspiration du patient.

Au bout d'un laps de temps, déterminée réglable, lorsque les compteurs 45 et 48 ont des signaux de sortie égaux (on verra plus loin une explication plus détaillée de leur fonctionnement), le comparateur 53 remet à zéro l'organe bistable 39 dont la sortie 40 passe au potentiel zéro, ce qui a pour effet immédiat, via le dispositif 41 d'assurer l'arrêt de la fourniture du gaz respirable (voie 5 obturée, voies 6 et 7 en communicaiton). Simultanément, le dispositif de temporisation 42 assure une commande retardée (de l'ordre de la seconde) du fonctionnement du dispositif de commande 43 de l'électrovanne 10 qui reste dans la même position, ainsi qu'une commande retardée du dispositif de mémorisation 35.

A la fin de la phase temporisée, la dispositif de temporisation 42 assure la commande du dispositif de mémorisation 33 (tension de référence mémorisée) et la commutation de l'électrovanne 10 qui assure la position d'attente d'un nouveau signal d'inspiration du patient (voies 9 et 11 en communication).

Le cycle tel qu'il vient d'être décrit peut alors se reproduire.

On détaille maintenant comment est déterminée la durée de la phase d'alimentation en gaz respiratoire, qui a lieu pendant une fraction de la phase inspiratoire du patient.

Pendant toute la phase de son alimentation en gaz respirable, lorsque la sortie 46 de l'organe bistable 39 est positive, le compteur 48 est en fonctionnement, tandis que le compteur 45 est à l'arrêt. Au début d'une phase d'inspiration du patient, l'organe bistable 39 bascule, la sortie 40 devient positive, et la sortie 46 revient au zéro. Le compteur 48 s'arrête, tandis que le compteur 45 se met en fonctionnement. Lorsque les signaux de sortie des compteurs 45 et 48 sont égaux, le comparateur 53 assure le basculement de l'organe bistable 39, de sorte que la sortie 40 revient à zéro (fermeture immédiate de l'électrovanne d'alimentation 4 et la sortie 46 devient positive, avec les conséquences rappelées plus haut.

On comprend que la durée d'alimentation en gaz respirable dépend de la durée de la phase de non fourniture de gaz respirable qui est directement liée à la durée de la phase expiratoire et, dans une certaine mesure, à la fréquence respiratoire du patient.

## Revendications

1. Procédé d'alimentation en oxygène respiratoire d'un patient, selon lequel on assure la dite alimentation seulement pendant une partie de la phase inspiratoire, qui est déclenchée par la détection d'une réduction de pression dans une chambre détectrice du type à baudruche et capteur opto-électronique, caractérisé en ce qu'on assure la détection opto-électronique par un capteur du genre à réflexion sur un réflecteur plaqué contre une paroi inférieure de baudruche qui est à l'opposé de la paroi de fixation de la dite baudruche, et en ce qu'on utilise la modification de courant du capteur lors d'une réduction de la distance capteur-baudruche due à une dilatation de la baudruche résultant d'une baisse de pression pour assurer une commande de l'alimentation en gaz respiratoire.

2. Procédé d'alimentation selon la revendication 1, caractérisé en ce qu'on assure, pendant chaque phase inspiratoire-expiratoire, une mémorisation d'une tension de référence de sortie de capteur représentative de la pression atmosphérique et on déclenche l'alimentation en gaz respirable dès le début d'une phase inspiratoire détectée par comparaison de cette tension de référence instantanée avec une tension représentative d'un dépression inspiratoire, ou fin de pression expiratoire.

3. Procédé d'alimentation selon la revendication 1 ou la revendication 2, caractérisé en ce qu'on règle la durée d'alimentation en gaz respirable en fonction directe de la durée de la phase expiratoire qui précède.

4. Procédé d'alimentation selon l'une quelconque des revendications 1 à 3, caractérisé en ce qu'on assure la mise à l'air libre du détecteur en vue de l'élaboration de la tension de référence un laps de temps après la fin d'une opération d'alimentation en oxygène respirable.

5. Dispositif d'alimentation en oxygène respirable, du genre comportant une conduite d'alimentation d'une lunette respiratoire ou d'une sonde trachéale, incorporant une électrovanne à trois voies, dont la troisième voie est raccordée à la troisième voie d'une électrovanne de mise à l'air atmosphérique d'un détecteur à capteur opto-électronique associe à une baudruche, des moyens de commande cyclique des dites vannes d'alimentation en oxygène respirable et de mise à l'air, caractérisé en ce que le capteur opto-électronique est du type à réflexion coopérant avec un réflecteur plaqué contre une paroi de baudruche opposée à une paroi de fixation de baudruche.

6. Dispositif d'alimentation selon la revendication 5, caractérisé en ce que les moyens de commande cyclique des vannes d'alimentation en oxygène respirable et de mise à l'air du détecteur comprennent un moyen d'élaboration d'une tension électronique de référence représentative de la pression dans la chambre du détecteur, un moyen de mémorisation de la dite tension de référence, des moyens de comparaison entre la tension de référence mémorisée et la tension instantanée représentative de la pression dans la chambre du détecteur, les dits moyens de comparaison assurant, lors d'une différence avec la tension de référence, la commande d'ouverture de la vanne d'alimentation en oxygène respirable et une commande différée d'ouverture de la vanne de mise à l'air libre du détecteur.

7. Dispositif d'alimentation selon la revendication 5 ou 6, caractérisé par des moyens de réglage de la durée d'alimentation en gaz respirable comprenant des moyens de comptage des durées d'alimentation et de non-alimentation en oxygène respiratoire et des moyens de comparaison des dites durées d'alimentation et des moyens de commande d'arrêt de l'électrovanne d'injection actifs sous l'action des dits moyens de comparaison de durées.

8. Installation d'alimentation mettant en oeuvre le dispositif selon une quelconque des revendications 5 à 7 caractérisée en ce qu'elle comporte une source de gaz respirable débitant dans un accumulateur-tampon à la sortie duquel est branchée la conduite d'alimentation de la lunette respiratoire ou sonde trachéale.

0285470

Office européen
des brevets

**RAPPORT DE RECHERCHE EUROPEENNE**

Numero de la demande

EP 88 40 0483

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | EP-A-0 183 593 (L'AIR LIQUIDE) * Figures 1,5; page 5, ligne 27 - page 6, ligne 14; page 9, lignes 15-28 * & FR-A-2 573 204 | 1,2 | A 61 M 16/00 G 01 L 9/00 |
| A | | 5 | |
| Y | WO-A-8 001 412 (VICTORIA UNIVERSITY OF MANCHESTER) * Figure 1; page 4, lignes 16-26 * | 1,2 | |
| A | | 5 | |
| Y | GB-A-2 178 193 (APPLIED MEMBRANE TECHN. INC.) * Figure 1; page 3, lignes 10-23,38-46; page 4, lignes 22-25 * | 2 | |
| A | | 3,5 | |
| A | EP-A-0 093 503 (SOPHISTICATED MECHANICAL EQUIPMENT) * Figure 1; résumé * | 1,5 | |
| A | WO-A-8 606 969 (ETELÄHÄMEEN KEUHKOVAMMAYHDISTYS R.Y.) * Figure 1; résumé * | 1,5 | |
| A | PATENT ABSTRACTS OF JAPAN, vol. 6, no. 229 (P-155)[1107], 16 novembre 1982; & JP-A-57 131 032 (SHIMAZU SEISAKUSHO K.K.) 13-08-1982 | 1,5 | |

DOMAINES TECHNIQUES RECHERCHES (Int. Cl.4)

A 61 M
G 01 L

Le présent rapport a été établi pour toutes les revendications

| Lieu de la recherche | Date d'achèvement de la recherche | Examinateur |
|---|---|---|
| LA HAYE | 08-06-1988 | JONES T.M. |